# FASCICULE DE BREVET EUROPEEN

(11) **EP 2 608 692 B1**
(45) Date de publication et mention de la délivrance du brevet: **19.08.2015**
(21) Numéro de dépôt: 11761657.3
(22) Date de dépôt: 23.08.2011
(51) Int. Cl.: B05B 11/00, B65D 83/30, A45D 34/04, A61M 35/00, B65D 83/20, B65D 83/40

(54) **TETE DE DISTRIBUTION DE PRODUIT FLUIDE**
SPENDERKOPF FÜR EIN FLÜSSIGES PRODUKT
FLUID PRODUCT DISPENSING HEAD

(30) Priorité: 26.08.2010 FR 1056794
(43) Date de publication de la demande: 03.07.2013
(73) Titulaire: Aptar France SAS, 27110 Le Neubourg (FR)
(72) Inventeur: DECOTTIGNIES, Laurent, F-95800 Cergy (FR); GOUDIGAN, Ludovic, F-27330 Epinay (FR)
(74) Mandataire: CAPRI
(86) Numéro de dépôt international: PCT/FR2011/051947
(87) Numéro de publication internationale: WO 2012/025691

(56) Documents cités:
- EP-A1- 1 842 799
- WO-A1-2009/025697
- FR-A1- 2 483 262
- FR-A1- 2 860 768
- FR-A1- 2 884 157

## Description

La présente invention concerne une tête de distribution de produit fluide destinée à être montée sur une tige d'actionnement axiale d'un organe de distribution, tel qu'une pompe. La tête comprend un noyau interne et une enveloppe externe, le noyau formant un manchon de raccordement d'axe X destiné à être monté sur la tige d'actionnement axiale, l'enveloppe formant un embout de distribution allongé qui est décalé par rapport à cet axe X. Une telle tête de distribution est notamment connue du document FR 2 860 768. Cette tête de distribution trouve une application privilégiée dans le domaine de la cosmétique, mais peut également être mise en oeuvre dans les domaines de la pharmacie et de la parfumerie. La présente invention concerne également un procédé de fabrication d'une telle tête de distribution.

Dans le document précité FR 2 860 768, le noyau forme une broche axiale insérée dans l'embout de distribution axial formé par l'enveloppe. Cette broche définit une rainure ouverte sur sa longueur qui est complétée par l'enveloppe pour former un canal d'alimentation reliant le manchon de raccordement du noyau à l'extrémité libre de l'embout de distribution de l'enveloppe. Le noyau est réalisé en une matière plastique rigide, de sorte que la broche n'est pas déformable. En revanche, il est prévu de réaliser l'enveloppe externe avec un matériau plastique relativement souple pour conférer une souplesse à l'extrémité libre de l'embout de distribution. Avec une telle configuration, le noyau est introduit axialement dans l'enveloppe, la broche du noyau étant déjà alignée avec l'embout de distribution de l'enveloppe. Ni le noyau, ni l'enveloppe ne subit de déformation marquée modifiant l'aspect général de la tête de distribution.

Cependant, cette tête de distribution souffre d'un inconvénient lié à la formation du canal d'alimentation reliant le manchon à l'extrémité libre de l'embout de distribution. Ce désavantage provient du fait que ce canal d'alimentation est formé par l'assemblage du noyau à l'intérieur de l'enveloppe, et nécessite de ce fait des contacts intimes étanches entre le noyau et l'enveloppe pour isoler parfaitement le canal d'alimentation sans risque de fuite. Théoriquement, cela semble possible, mais en pratique, il s'est avéré qu'il est impossible d'isoler parfaitement ce canal d'alimentation. En effet, on a constaté des fuites de produit fluide entre le noyau et l'enveloppe. Le produit fluide qui a fui se détériore et contamine ensuite le produit fluide distribué à travers le canal d'alimentation. Par conséquent, la configuration du canal d'alimentation de cette tête de distribution de l'art antérieur ne garantit pas une parfaite conservation du produit fluide distribué. Ceci est un inconvénient majeur, notamment avec des produits fluides particulièrement délicats.

D'autre part, la conception de la tête de distribution du document FR 2 860 768 oblige à utiliser, pour l'enveloppe, des matériaux qui sont compatibles avec le produit fluide à distribuer, étant donné que le canal d'alimentation est formé à la fois par le noyau et l'enveloppe. Cela réduit considérablement le choix pour le matériau constitutif de l'enveloppe, qui doit en outre conférer un attrait esthétique, puisque visible.

L'invention a pour but de remédier aux inconvénients précités de l'art antérieur en définissant une nouvelle conception de tête de distribution de ce type qui élimine tout risque de fuite de produit fluide au niveau du canal d'alimentation reliant le manchon de distribution à l'orifice de distribution de l'embout de l'enveloppe.

Pour ce faire, la présente invention prévoit une tête de distribution selon la revendication 1.

La canule déformable peut être réalisée de manière monobloc avec le reste du noyau, ou en variante, la canule déformable peut être rapportée ou surmoulée sur le noyau. Contrairement à l'art antérieur précité, la canule qui relie le manchon de raccordement à l'orifice de distribution est uniquement formé par le noyau, et non pas par l'assemblage du noyau et de l'enveloppe. Ainsi, l'enveloppe peut être réalisée avec n'importe quel matériau, et même un matériau qui est incompatible avec le produit fluide à distribuer. D'autre part, tout risque de fuite le long de la canule est ainsi éliminé. En outre, en réalisant la canule de manière rectiligne, cela permet de réaliser le noyau par injection-moulage avec des éléments de moule relativement simples. L'introduction du noyau dans l'enveloppe se fait de la même manière que dans l'art antérieur, à l'exception de la canule qui est déformée lors de cette opération.

Selon une forme de réalisation pratique, la canule peut présenter une épaisseur de paroi inférieure à celle du manchon de raccordement pour lui conférer une souplesse. La canule peut même présenter des épaisseurs de paroi différentes sur sa longueur. La seconde section coudée peut par exemple présenter une épaisseur de paroi inférieure à celle des deux autres sections qui ne sont pas déformées. On peut également imaginer de réaliser la canule avec un matériau plus souple que le restant du noyau.

Selon un autre aspect de la présente invention, la canule peut s'étendre sensiblement sur toute la longueur de l'embout. Ainsi, il n'y a pas de produit fluide qui s'écoule en contact de l'embout de distribution, la canule assurant à elle seule l'alimentation du produit fluide depuis le manchon de raccordement jusqu'à l'orifice de distribution. A cet effet, l'orifice de distribution peut être formé par la canule, ou en variante, l'enveloppe peut former l'orifice de distribution.

Selon un mode de réalisation avantageux, l'enveloppe est réalisée en métal, tel que du zamak ou de l'acier, pour conférer une sensation de froid au contact de la peau. Il s'agit là d'une conception particulièrement originale, étant donné que l'utilisateur pense que le produit fluide est alimenté à travers la tête de distribution en métal, alors qu'en réalité le produit fluide est alimenté à travers la canule en matière plastique du noyau. On évite ainsi tout risque d'oxydation ou de détérioration du produit fluide en contact du métal. On peut même utiliser les matériaux ou traitements qui sont incompatibles avec le produit fluide à distribuer.

Selon une forme de réalisation pratique, la canule comprend une première section sensiblement rectiligne transversale à l'axe X, une seconde section coudée et une troisième section insérée dans l'embout de distribution. Avantageusement, l'embout peut s'étendre parallèlement à l'axe X selon un axe Y, la canule déformable s'étend sensiblement de l'axe X jusqu'à l'axe Y, la troisième section est sensiblement rectiligne et s'étend sur l'axe Y.

Selon un autre aspect de l'invention, la première section sensiblement rectiligne peut être oblique par rapport à l'axe X. Ainsi, la première section permet d'écarter la canule de l'axe X, la seconde section permet de ramener la canule parallèlement à l'axe X, et la troisième section s'étend simplement parallèlement à l'axe X selon l'axe Y. La canule n'a été déformée de manière conséquente qu'au niveau de sa seconde section.

Selon une forme de réalisation intéressante, l'enveloppe peut définir une surface d'appui qui s'étend en aval du manchon de raccordement en coupant l'axe X. Il s'agit là d'une caractéristique que l'on trouve également dans le document de l'art antérieur précité.

Avantageusement, le noyau forme une couronne qui est reçue fixement dans une jupe de l'enveloppe.

L'invention définit également un procédé de fabrication d'une tête de distribution de produit fluide destinée à être montée sur une tige d'actionnement axiale d'un organe de distribution, tel qu'une pompe, la tête comprenant un noyau interne et une enveloppe externe, le noyau formant un manchon de raccordement d'axe X destiné à être monté sur la tige d'actionnement axiale, l'enveloppe formant un embout de distribution allongé qui est décalé par rapport à cet axe X, le procédé étant caractérisé en ce qu'il prévoit de réaliser le noyau avec une canule oblique rectiligne déformable, d'engager la canule dans l'embout, de couder la canule et d'introduire à fond le noyau dans l'enveloppe. L'opération de montage n'est pas plus compliquée que celle de la tête de distribution du document FR 2 860 768, la seule différence étant que la canule est déformée lors du montage. Cependant, cela ne génère pas d'opération ou de manipulation supplémentaire.

L'esprit de l'invention réside dans le fait que le noyau forme à lui seul le passage d'alimentation de produit fluide reliant le manchon de raccordement à l'orifice de distribution. L'enveloppe ne sert que d'habillage et ne vient pas en contact du produit fluide, excepté au niveau de l'orifice de distribution éventuellement. Sans compliquer ni le moulage, ni le montage, on obtient une tête de distribution qui s'affranchit de tout risque de fuite interne.

L'invention sera maintenant plus amplement décrite en référence aux dessins joints, donnant à titre d'exemple non limitatif un mode de réalisation de l'invention.

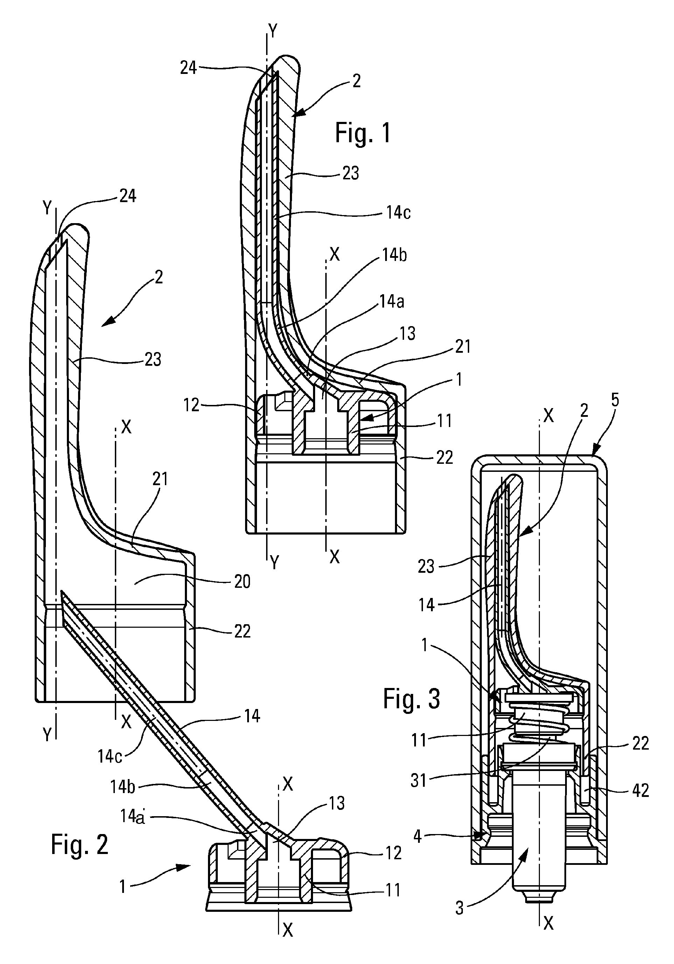

Sur les figures :
La figue 1 est une vue en coupe transversale verticale à travers une tête de distribution de l'invention à l'état monté,
Le figure 2 est une vue en coupe transversale éclatée montrant la tête de distribution en cours de montage,
La figure 3 est une vue de la tête de distribution des figures 1 et 2, associée à un organe de distribution.

On se référera indifféremment aux figures 1 à 3 pour expliquer la structure et le montage d'une tête de distribution réalisée selon une forme de réalisation non limitative de l'invention. La tête de distribution comprend deux éléments constitutifs essentiels, à savoir un noyau interne 1 et une enveloppe externe 2, le noyau 1 étant engagé et reçu de manière fixe et définitive à l'intérieur de l'enveloppe externe 2, comme visible sur la figure 1. Une fois la tête de distribution montée sur un organe de distribution, comme représenté sur la figure 3, seule l'enveloppe externe 2 est visible par l'utilisateur, le noyau interne 1 étant entièrement masqué.

Le noyau interne 1 est une pièce, de préférence monobloc, qui peut être réalisée par injection-moulage de matière plastique appropriée. En variante, il peut également être réalisé par assemblage de plusieurs pièces séparées. On peut également envisager de réaliser le noyau avec des matières plastique différentes selon une technique de surmoulage, de co-moulage ou de bi-injection. Le noyau interne 1 comprend un manchon de raccordement 11 qui s'étend le long d'un axe X. Ce manchon de raccordement 11 est destiné à être monté sur une tige d'actionnement 31 d'un organe de distribution 3, tel qu'une pompe ou une valve. En général, le manchon de raccordement 11 est simplement emmanché en force sur l'extrémité libre de la tige d'actionnement 31. Cette tige d'actionnement 31 est creuse et sert de conduit de sortie de produit fluide pour l'organe de distribution 3. Le conduit de raccordement 11 est bien entendu creux et forme à son extrémité supérieure une chambre de liaison 13 qui est également située sur l'axe X. Le noyau interne 1 comprend également une couronne périphérique 12 qui s'étend concentriquement autour du manchon de raccordement 11. La couronne 12 rejoint le manchon 11 au niveau de l'extrémité supérieure du manchon 11 autour de la chambre de liaison 13. Cette chambre 13 communique directement avec une canule d'alimentation déformable 14 qui est initialement rectiligne, comme visible sur la figure 2 et finalement coudée, comme visible sur les figures 1 et 3. La canule 14 a donc été déformée de sa condition initiale rectiligne dans sa condition finale coudée lors du montage du noyau 1 dans l'enveloppe 2. Il faut également remarquer que l'orientation initiale de la canule 14, comme visible sur la figure 2 est oblique par rapport à l'axe X. La canule 14 s'étend à partir de la chambre de liaison 13 qui est sur l'axe X, et ensuite de manière transversale vers le haut en éloignant l'axe X. Sur la figure 2, la canule est rectiligne, de sorte que son extrémité libre est à une distance maximale de l'axe X, alors que sur la figure 1, la canule 14 a été déformée, de sorte que son extrémité libre s'est rapprochée de l'axe X, sans toutefois revenir à l'axe X. On peut ainsi définir la canule comme ayant trois sections différentes, à savoir une première section 14a qui est directement reliée au manchon de raccordement 11 et à la chambre de transition 13, une seconde section 14b reliée à la première section 14a, et qui va subir la déformation, et enfin une troisième section 14c dont l'orientation a été modifiée, mais qui est restée rectiligne. Ainsi, la canule comprend deux sections rectilignes qui restent non déformées, à savoir les première et troisième sections 14a et 14c, ainsi qu'une section intermédiaire 14b qui va subir la déformation pour permettre le changement d'orientation de la troisième section rectiligne 14c. En comparant le noyau 1 des figures 2 et 1, on peut clairement remarquer que la première section 14a n'a subi aucune déformation, que la section 14b a été coudée et que la troisième section 14c n'a pas été déformée, mais son orientation a été changée. Au final, comme représenté sur la figure 1, la troisième section 14c s'étend parallèlement à l'axe X, le long d'un axe Y qui est décalé par rapport à l'axe X.

La déformation de la canule 14 du noyau interne 1 est induite et imposée par l'enveloppe externe 2. En effet, cette enveloppe 2 comprend une jupe sensiblement cylindrique 22 qui définit un logement interne 20 pour le noyau 1. La jupe cylindrique 22 se raccorde à son extrémité supérieure à une surface d'appui 21 qui coupe l'axe X. L'utilisateur va appuyer, à l'aide d'un ou plusieurs doigts, sur cette surface d'appui 21 pour déplacer axialement la tête de distribution selon l'axe X, afin d'actionner l'organe de distribution 3. La jupe 22 et la surface d'appui 21 se raccordent à un embout de distribution allongé 23 qui s'étend le long de l'axe Y, qui est parallèle à l'axe X, mais décalé par rapport à celui-ci. L'embout de distribution 23 peut par exemple s'étendre de long d'un côté de la jupe 22. On peut dire que l'embout de distribution 23 est excentré ou désaxé par rapport à l'axe de symétrie constitué par l'axe X. A son extrémité supérieure, l'embout de distribution 23 définit un orifice de distribution 24 qui est ici situé sur l'axe Y. On peut également envisager de réaliser l'orifice de distribution 24 de manière latérale sur la hauteur de l'embout de distribution 23, à proximité de son extrémité supérieure libre. L'enveloppe externe 2 peut être réalisée en n'importe quel matériau. Mais de préférence, l'enveloppe externe est réalisée en métal, par exemple du zamak ou de l'acier, pour conférer une sensation de froid au contact de la peau de l'utilisateur. Ceci peut être particulièrement avantageux lorsque le produit fluide distribué est destiné à avoir un effet apaisant pour la peau de l'utilisateur. Dans ce cas, la sensation de froid procurée par l'enveloppe améliore encore cet effet apaisant. En outre, étant donné que le produit fluide ne vient pas (ou pratiquement pas) en contact de l'enveloppe, on peut même utiliser les matériaux (métal ou autres) ou traitements (par exemple la galvanisation) qui sont incompatibles avec le produit fluide à distribuer.

Le noyau interne 1 est introduit dans l'enveloppe externe 2, comme représenté sur la figure 2. La canule 14 encore rectiligne est engagée à l'intérieur de la jupe 22 de l'enveloppe. En continuant à insérer le noyau 1 dans l'enveloppe 2, l'extrémité libre de la canule 14 va s'engager à l'intérieur de l'embout de distribution 23. La canule 14 va alors commencer à être déformée. En continuant l'insertion du noyau 1 dans l'enveloppe 2, la couronne 12 du noyau s'engage à l'intérieur de la jupe 22 de l'enveloppe. La canule 14 est alors déjà bien déformée. Finalement, le noyau 1 est reçu à fond à l'intérieur de l'enveloppe 2, comme représenté sur la figure 1. La couronne 12 est de préférence encliquetée à l'intérieur de la jupe 22. La surface d'appui 21 s'étend juste en aval du manchon de raccordement 11 en coupant l'axe X. Quant à la canule 14, elle a été déformée de manière à ce que son extrémité libre vienne en contact intime avec l'orifice de distribution 24 de l'enveloppe 2. La première section 14a n'a pas subi de déformation, de même que la troisième section 14c, dont seule l'orientation a été modifiée. En revanche, la seconde section intermédiaire 14b a été coudée pour se conformer à la configuration interne de l'enveloppe 2. On peut dire que la seconde section 14b remplit une fonction de jonction souple déformable entre les deux sections 14a et 14c qui sont restées non déformées.

Grâce à cette canule déformable 14, le produit fluide peut être acheminé du manchon de raccordement 11 jusqu'à l'orifice de distribution 24 sans jamais venir en contact avec l'enveloppe externe 2. Tout risque de fuite le long de cet acheminement est évité, et tout risque de détérioration du produit fluide en contact de l'enveloppe externe 2 est également évité. Dans le mode de réalisation représenté sur les dessins, l'orifice de distribution 24 est formé par l'enveloppe 2, mais on peut également imaginer que la canule s'étende jusqu'à la surface de l'enveloppe 2 de manière à former l'orifice de distribution.

En se référant à la figure 3, on peut voir que la tête de distribution de l'invention peut parfaitement s'intégrer à un organe de distribution classique 3, qui peut être une pompe ou une valve. Le manchon à raccordement 11 est emmanché sur la tige d'actionnement 31 qui est déplaçable axialement en va-et-vient le long de l'axe X. L'organe de distribution est pourvu d'une bague de fixation 4 destinée au montage fixe et étanche sur un col de réservoir (non représenté). On peut remarquer que l'extrémité inférieure de la jupe 22 de l'enveloppe 2 est insérée dans une gorge annulaire 42 formée par la bague de fixation 4. Ainsi, le noyau interne 1 est complètement masqué, seule l'enveloppe externe 22 est visible par l'utilisateur. Il a l'impression que la tête de distribution est uniquement formée par l'enveloppe externe 2 : ceci est encore renforcé par le fait que l'extrémité libre de la canule 14 ne forme pas l'orifice de distribution 24. Lorsque l'enveloppe externe 2 est réalisée en métal, l'utilisateur peut penser que le produit fluide est acheminé directement en contact du métal. Optionnellement, la tête de distribution peut être coiffée d'un capot de protection 5 monté sur la bague de fixation 4.

Dans le mode de réalisation utilisé pour illustrer la présente invention, la canule 14 reste non déformée au niveau de sa troisième section 14c. On peut toutefois envisager dans le cadre de la présente invention que cette troisième section 14c soit également déformée lors de son insertion dans l'enveloppe externe 2. On peut également imaginer que l'embout de distribution 23 ne s'étende pas parallèlement à l'axe X : il peut être oblique, ou même coudé.

Grâce à l'invention, on dispose d'une tête de distribution originale à embout de distribution allongé décalé dont l'alimentation en produit fluide est uniquement assurée par le noyau interne 1.

## Revendications

1. Tête de distribution de produit fluide destinée à être montée sur une tige d'actionnement axiale (31) d'un organe de distribution (3), tel qu'une pompe, la tête comprenant un noyau interne (1) et une enveloppe externe (2), le noyau (1) formant un manchon de raccordement (11) d'axe (X) destiné à être monté sur la tige d'actionnement axiale (31), l'enveloppe (2) formant un embout de distribution allongé (23) qui est décalé par rapport à cet axe (X), **caractérisée en ce que** le noyau (1) forme une canule déformable (14) qui est reliée au manchon de raccordement (11) et qui s'étend dans l'embout (23) de l'enveloppe (2), la canule (14) étant initialement rectiligne sur toute sa longueur et oblique par rapport à l'axe (X), la canule (14) étant ensuite déformée lors de son introduction dans l'embout (23).

2. Tête de distribution selon la revendication 1, dans laquelle la canule (14) présente une épaisseur de paroi inférieure à celle du manchon de raccordement (11) pour lui conférer une souplesse.

3. Tête de distribution selon la revendication 1 ou 2, dans laquelle la canule (14) s'étend sensiblement sur toute la longueur de l'embout (23).

4. Tête de distribution selon l'une quelconque des revendications précédentes, dans laquelle l'enveloppe (2) forme un orifice de distribution (24).

5. Tête de distribution selon l'une quelconque des revendications précédentes, dans laquelle l'enveloppe (2) est réalisé en métal, tel que du zamak ou de l'acier, pour conférer une sensation de froid au contact de la peau.

6. Tête de distribution selon l'une quelconque des revendications précédentes, dans laquelle la canule (14) comprend une première section sensiblement rectiligne (14a) transversale à l'axe X, une seconde section coudée (14b) et une troisième section (14c) insérée dans l'embout de distribution (23).

7. Tête de distribution selon la revendication 6, dans laquelle l'embout s'étend parallèlement à l'axe X selon un axe Y, la canule déformable s'étend sensiblement de l'axe X jusqu'à l'axe Y, la troisième section (14c) est sensiblement rectiligne et s'étend sur l'axe Y.

8. Tête de distribution selon la revendication 6, dans laquelle la première section sensiblement rectiligne (14a) est oblique par rapport à l'axe X.

9. Tête de distribution selon l'une quelconque des revendications précédentes, dans laquelle l'enveloppe (2) définit une surface d'appui (21) qui s'étend en aval du manchon de raccordement en coupant l'axe X.

10. Tête de distribution selon l'une quelconque des revendications précédentes, dans laquelle le noyau (1) forme une couronne (12) qui est reçue fixement dans une jupe (22) de l'enveloppe (2).

11. Procédé de fabrication d'une tête de distribution de produit fluide destinée à être montée sur une tige d'actionnement axiale (41) d'un organe de distribution (3), tel qu'une pompe, la tête comprenant un noyau interne (1) et une enveloppe externe (2), le noyau (1) formant un manchon de raccordement (11) d'axe (X) destiné à être monté sur la tige d'actionnement axiale (31), l'enveloppe (2) formant un embout de distribution allongé (23) qui est décalé par rapport à cet axe (X), le procédé étant **caractérisé en ce qu'**il prévoit de réaliser le noyau (1) avec une canule oblique rectiligne déformable (14), d'engager la canule (14) dans l'embout (23), de couder la canule (14) et d'introduire à fond le noyau (1) dans l'enveloppe (2).

## Patentansprüche

1. Spenderkopf für ein fluides Produkt zur Montage auf einer axialen Betätigungsstange (31) einer Spendereinrichtung (3), wie einer Pumpe, wobei der Kopf einen internen Kern (1) und eine externe Hülle (2) umfasst, wobei der Kern (1) eine Verbindungsmuffe (11) der Achse (X) zur Montage auf der axialen Betätigungsstange (31) bildet, wobei die Hülle (2) einen langgezogenen Spenderansatz (23) bildet, der in Bezug auf diese Achse (X) versetzt ist, **dadurch gekennzeichnet, dass** der Kern (1) eine verformbare Kanüle (14) bildet, die mit der Verbindungsmuffe (11) verbunden ist und sich in dem Ansatz (23) der Hülle (2) erstreckt, wobei die Kanüle (14) anfangs auf ihrer gesamten Länge geradlinig und in Bezug auf die Achse (X) schräg ist, wobei die Kanüle (14) anschließend bei ihrer Einführung in den Ansatz (23) verformt wird.

2. Spenderkopf nach Anspruch 1, wobei die Kanüle (14) eine Wandstärke aufweist, die kleiner ist als diejenige der Verbindungsmuffe (11), um ihr eine Flexibilität zu verleihen.

3. Spenderkopf nach Anspruch 1 oder 2, wobei sich die Kanüle (14) im Wesentlichen auf der gesamten Länge des Ansatzes (23) erstreckt.

4. Spenderkopf nach einem der vorhergehenden Ansprüche, wobei die Hülle (2) eine Spenderöffnung (24) bildet.

5. Spenderkopf nach einem der vorhergehenden Ansprüche, wobei die Hülle (2) aus Metall, wie Zamak oder Stahl, gefertigt ist, um beim Kontakt mit der Haut ein Kältegefühl zu erzeugen.

6. Spenderkopf nach einem der vorhergehenden Ansprüche, wobei die Kanüle (14) einen ersten, im Wesentlichen geradlinigen Abschnitt (14a) quer zur Achse X, einen zweiten, gebogenen Abschnitt (14b) sowie einen dritten Abschnitt (14c), der in den Spenderansatz (23) eingefügt ist, umfasst.

7. Spenderkopf nach Anspruch 6, wobei sich der Ansatz parallel zur Achse X entlang einer Achse Y erstreckt, wobei sich die verformbare Kanüle im Wesentlichen von der Achse X bis zur Achse Y erstreckt, wobei der dritte Abschnitt (14c) im Wesentlichen geradlinig ist und sich zur Achse Y erstreckt.

8. Spenderkopf nach Anspruch 6, wobei der erste, im Wesentlichen geradlinige Abschnitt (14a) in Bezug auf die Achse X schräg ist.

9. Spenderkopf nach einem der vorhergehenden Ansprüche, wobei die Hülle (2) eine Stützfläche (21) definiert, die sich von der Verbindungsmuffe weg erstreckt und dabei die Achse X schneidet.

10. Spenderkopf nach einem der vorhergehenden Ansprüche, wobei der Kern (1) einen Kranz (12) bildet, der fest in einem Mantel (22) der Hülle (2) aufgenommen ist.

11. Verfahren zur Herstellung eines Spenderkopfes für ein fluides Produkt zur Montage auf einer axialen Betätigungsstange (31) einer Spendereinrichtung (3), wie einer Pumpe, wobei der Kopf einen internen Kern (1) und eine externe Hülle (2) umfasst, wobei der Kern (1) eine Verbindungsmuffe (11) der Achse (X) zur Montage auf der axialen Betätigungsstange (31) bildet, wobei die Hülle (2) einen langgezogenen Spenderansatz (23) bildet, der in Bezug auf diese Achse (X) versetzt ist, wobei das Verfahren **dadurch gekennzeichnet ist, dass** es vorsieht, den Kern (1) mit einer verformbaren, geradlinigen schrägen Kanüle (14) zu verwirklichen, die Kanüle (14) in den Ansatz (23) einzubringen, die Kanüle (14) zu biegen und sie am Boden des Kerns (1) in die Hülle (2) einzuführen.

## Claims

1. A fluid dispenser head for mounting on an axial actuator rod (31) of a dispenser member (3), such as a pump, the head comprising an inner core (1) and an outer casing (2), the core (1) forming a connection sleeve (11) of axis (X) for mounting on the axial actuator rod (31), the casing (2) forming a dispenser endpiece (23) that is elongate and that is offset relative to the axis (X), the dispenser head being **characterized in that** the core (1) forms a deformable cannula (14) that is connected to the connection sleeve (11) and that extends in the endpiece (23) of the casing (2), the cannula (14) initially being rectilinear over its entire length and sloping relative to the axis (X), the cannula then being deformed while it is being inserted into the endpiece (23).

2. A dispenser head according to claim 1, wherein the cannula (14) presents a wall thickness that is less than the wall thickness of the connection sleeve (11) so as to impart flexibility thereto.

3. A dispenser head according to claim 1 or claim 2, wherein the cannula (14) extends over substantially the entire length of the endpiece (23).

4. A dispenser head according to any preceding claim, wherein the casing (2) forms a dispenser orifice (24).

5. A dispenser head according to any preceding claim, wherein the casing (2) is made out of metal, e.g. zamak or steel, so as to impart a cold sensation on contact with the skin.

6. A dispenser head according to any preceding claim, wherein the cannula (14) includes a first section (14a) that is substantially rectilinear and that extends transversely relative to the axis X, a second section (14b) that is bent, and a third section (14c) that is inserted into the dispenser endpiece (23).

7. A dispenser head according to claim 6, wherein the endpiece extends parallel to the axis X along an axis Y, the deformable cannula extends substantially from the axis X to the axis Y, the third section (14c) is substantially rectilinear and extends along the axis Y.

8. A dispenser head according to claim 6, wherein the substantially-rectilinear first section (14a) slopes relative to the axis X.

9. A dispenser head according to any preceding claim, wherein the casing (2) defines a bearing surface (21) that extends downstream from the connection sleeve, intersecting the axis X.

10. A dispenser head according to any preceding claim, wherein the core (1) forms a collar (12) that is received in stationary manner in a skirt (22) of the casing (2).

11. A method of manufacturing a fluid dispenser head for mounting on an axial actuator rod (31) of a dispenser member (3), such as a pump, the head comprising an inner core (1) and an outer casing (2), the core (1) forming a connection sleeve (11) of axis (X) for mounting on the axial actuator rod (31), the casing (2) forming a dispenser endpiece (23) that is elongate and that is offset relative to the axis (X), the method being **characterized in that** provision is made to make the core (1) with a deformable, rectilinear, sloping cannula (14), to engage the cannula (14) in the endpiece (23), to bend the cannula (14), and to insert the core (1) fully into the casing (2).
